# EUROPÄISCHE PATENTSCHRIFT

(11) **EP 2 882 468 B1**
(45) Veröffentlichungstag und Bekanntmachung des Hinweises auf die Patenterteilung: **06.09.2017**
(21) Anmeldenummer: 13737203.3
(22) Anmeldetag: 10.07.2013
(51) Int. Cl.: A61M 1/00

(54) **ANSCHLUSSVORRICHTUNG ZUR VERWENDUNG BEI DER UNTERDRUCKTHERAPIE VON WUNDEN**
CONNECTION DEVICE FOR USE IN NEGATIVE PRESSURE THERAPY FOR WOUNDS
DISPOSITIF DE RACCORDEMENT DESTINÉ À ÊTRE UTILISÉ DANS LE TRAITEMENT DE PLAIES PAR PRESSION NÉGATIVE

(30) Priorität: 09.08.2012 DE 102012214178
(43) Veröffentlichungstag der Anmeldung: 17.06.2015
(73) Patentinhaber: Paul Hartmann AG, 89522 Heidenheim (DE)
(72) Erfinder: ECKSTEIN, Axel, 89522 Heidenheim (DE); CROIZAT, Pierre, 89542 Hebrechtingen (DE); SAGAS, Ali, 15235 Kfar-Kama (IL); STEIN, Oded, 16960 Kfar Hahoresh (IL)
(74) Vertreter: DREISS Patentanwälte PartG mbB
(86) Internationale Anmeldenummer: PCT/EP2013/064526
(87) Internationale Veröffentlichungsnummer: WO 2014/023501

(56) Entgegenhaltungen:
- EP-A2- 1 129 734
- DE-A1-102010 006 272
- US-A1- 2006 100 586
- US-A1- 2010 063 464

## Beschreibung

Die Erfindung betrifft eine Anschlussvorrichtung zur Verwendung bei der Unterdrucktherapie von Wunden, mit einem mit Unterdruck und/oder fluiden Medien beaufschlagbaren biegsamen Leitungsmittel mit wenigstens zwei Lumen und mit einem mit dem Leitungsmittel verbundenen Kopplungskörper, wobei der Kopplungskörper auf einen die Wunde überfangenden und gegen die Atmosphäre dichtend abschließenden Unterdruckverband aufbringbar ist, wobei das Leitungsmittel durch wenigstens eine Öffnung typischerweise in einer dem Unterdruckverband zugewandten Wandung des Kopplungskörpers und durch wenigstens eine Öffnung in dem Unterdruckverband hindurch mit dem Wundraum kommuniziert, wobei der Kopplungskörper aus einem elastomeren biegsamen Material ausgebildet ist.

In der jüngeren Vergangenheit hat die Unterdruckbehandlung von Wunden, insbesondere von problematisch heilenden Wunden, eine zunehmende Bedeutung erlangt. Dabei bedeutet Unterdruckbehandlung, dass ein an sich der umgebenden Atmosphäre ausgesetzter Körper- oder Wundbereich durch noch näher zu beschreibende Mittel druckdicht bzw. unterdruckdicht gegen die Umgebung, also die Atmosphäre in der wir leben und atmen, abgeschlossen wird, wobei innerhalb des abgeschlossenen Wundbereichs auf ebenfalls noch zu erläuternde Weise ein gegenüber dem Atmosphärendruck verringerter Druck, mithin also Unterdruck relativ zur Atmosphäre angelegt und dauerhaft aufrechterhalten werden kann. Wenn auf dem hier in Rede stehenden Gebiet von Unterdruck die Rede ist, so wird hiermit ein Druckbereich verstanden, der typischerweise zwischen 0 und 250 mmHg (mm Quecksilbersäule) unterhalb des umgebenden Atmosphärendrucks liegt. Es hat sich gezeigt, dass dies der Wundheilung förderlich ist. Für den unterdruckdichten Abschluss wird ein Unterdruckverband vorgesehen, der beispielsweise eine druck- bzw. unterdruckdichte Folienschicht umfassen kann, die typischerweise mit einem die Wunde umgebenden unversehrten Körperbereich verklebt ist, so dass auf diese Weise ein dichter Abschluss erreicht werden kann. Um ausgehend von einer Unterdruck erzeugenden Einrichtung, also einer Unterdruckpumpe im weitesten Sinn, einen Unterdruck an den Wundraum heranzuführen und dort aufrechtzuerhalten, können bei hier in Rede stehenden Systemen zur Unterdrucktherapie von Wunden mit Unterdruck beaufschlagbare Leitungsmittel verwendet werden, die mittels einer Anschlussvorrichtung mit dem Unterdruckverband zusammenwirken, um Unterdruck an bzw. in den Wundraum zu bringen.

Aus DE 10 2009 060 596 A1 und US 2006/0100586 A1 ist eine Anschlussvorrichtung der eingangs erwähnten Art bekannt. Das Leitungsmittel ist dabei auf die wundabgewandte Seite eines Kopplungskörpers aufgebracht und dort fixiert. Zur Unterdruckkommunikation mit dem Wundraum werden Öffnungen vorgesehen, die sich durch die Wandung des Leitungsmittels und des Kopplungskörpers hindurch erstrecken. Eine ähnliche Anschlussvorrichtung ist aus DE 10 2010 006 272 A1 vorbekannt, bei der das Leitungsmittel selbst einen Kopplungskörper bildet, indem ein wundseitiger Längsendabschnitt des Leitungsmittels beidseits einstückig in seitliche Flügelabschnitte übergeht. Die einstückige Ausbildung, also die Herstellung von Leitungsmittel und Kopplungskörper in einem einzigen Herstellungsvorgang erweist sich jedoch als extremst kompliziert und daher nachteilig. Mit US 2010/0063464 wurde vorgeschlagen, einen in die Wunde einlegbaren Druckverteilungskörper einstückig zu spritzen.

Weitere Anschlussvorrichtungen sind vorbekannt aus DE 10 2010 006 273 A1. Bei dieser Ausführungsform wird das Leitungsmittel sandwichartig zwischen verschiedene Schichten, die einen Kopplungskörper zum Wundverband hin bilden, angeordnet und dichtend fixiert, was ebenfalls als aufwändig anzusehen ist.

Mit WO 2009/124548 A1 wird vorgeschlagen, einen eher tassen- oder tellerförmigen Kopplungskörper mit radial erstreckten Anlagebereichen und mit einem Aufnahmeabschnitt zum Einstecken eines Leitungsmittels einstückig im Spritzgießverfahren herzustellen. EP 1 129 734 A2 verwendet eine Kappe zum Abschluss eines in eine Wunde einlegbaren Leitu ngsm ittels.

Der vorliegenden Erfindung liegt die Aufgabe zugrunde, eine Anschlussvorrichtung zu schaffen, die prozesssicher und auf wirtschaftliche Weise herstellbar ist und die verhältnismäßig flach ausgebildet werden kann.

Diese Aufgabe wird durch bei eine Anschlussvorrichtung mit den Merkmalen des Anspruchs 1 gelöst.

Das vorzugsweise flach bauende Leitungsmittel der erfindungsgemäßen Anschlussvorrichtung wird zunächst separat hergestellt, was den grundsätzlichen Vorteil der Möglichkeit einer endlosen Fertigung, vorzugsweise durch Extrusion, mit sich bringt, wobei im Anschluss daran Abschnitte einer jeweils gewünschten Leitungslänge durch Ablängung bereitgestellt werden können. Der Kopplungskörper wird dann durch Anspritzen an einen Endabschnitt des Leitungsmittels gebildet und durch Anfügen und Befestigen der Abschlusskappe komplettiert. Es erweist sich als besonders vorteilhaft, dass bei dieser Art der Herstellung des Kopplungskörpers zugleich ein dichtender Übergang zu dem Leitungsmittel hin prozesssicher ausgebildet werden kann, oder anders ausgedrückt, dass im Zuge der Bildung des Kopplungskörpers zugleich das zugehörige und bereitgestellte Leitungsmittel dichtend an den Kopplungskörper angefügt wird.

Es sei auch erwähnt, dass die wenigstens eine Öffnung in der dem Unterdruckverband zugewandten Wandung des Kopplungskörpers entweder im Zuge des Spritzgießvorgangs durch geeignete Ausbildung des Spritzgießwerkzeugs hergestellt werden kann oder in einem nachfolgenden Arbeitsgang, beispielsweise durch einen Stanzvorgang, ausgebildet werden kann. Der Herstellung im Zuge des Spritzgießvorgangs wird indessen der Vorzug gegeben. Vorzugsweise werden mehrere Öffnungen vorgesehen, die vorzugsweise langlochförmig ausgebildet sind.

Es erweist sich als vorteilhaft, wenn das Leitungsmittel im Wesentlichen parallel zu der Ebene der flächenhaften Erstreckung des Kopplungskörpers an den Kopplungskörper herangeführt ist. In diesem Zusammenhang wird unter "im Wesentlichen parallel" eine Neigung zu dieser Erstreckungsebene, die auch die Anlagefläche an den Unterdruckverband bildet, von bis zu 15° verstanden.

Es erweist sich weiter als vorteilhaft, wenn der Kopplungskörper so ausgebildet ist, dass nach dem Anspritzen wenigstens ein Lumen frei ausmündet, und dass durch Befestigen der Abschlusskappe das frei ausmündende Lumen nach außen dicht abgeschlossen ist. Es erweist sich bei dieser Variante als vorteilhaft, dass im Zuge des Anspritzens des den Kopplungskörper bildenden elastomeren Materials an den Endabschnitt des Leitungsmittels Fluidleitungsabschnitte, die nachfolgend mit "Lumen" bezeichnet werden, innerhalb des Kopplungskörpers gebildet werden. Dies wird prozesstechnisch dadurch realisiert, dass innerhalb des Spritzgießwerkzeugs beispielsweise stangenförmige Elemente vorgesehen sind, die dann diese Lumen bilden und insbesondere verhindern, dass beim Spritzgießvorgang Material in die Lumen des Leitungsmittels gelangt. Es wäre aber auch denkbar, dass sich das Leitungsmittel bzw. der Endabschnitt des Leitungsmittels bis zu der Innenseite der Abschlusskappe erstreckt und dessen Lumen dann durch die Abschlusskappe dichtend verschlossen werden.

Erfindungsgemäß ist der Kopplungskörper so ausgebildet, dass nach dem Anspritzen wenigstens zwei Lumen frei ausmünden und durch Befestigen der Abschlusskappe die frei ausmündenden Lumen nach außen dicht abgeschlossen und miteinander durchströmbar verbunden werden, indem die Abschlusskappe eine die wenigstens zwei Lumen miteinander verbindende Ausnehmung aufweist. Dies bringt den Vorteil mit sich, dass die Lumen des Leitungsmittels bzw. des Kopplungskörpers an ihren distalen Enden und damit gewissermaßen totraumfrei miteinander verbunden werden.

Dies erweist sich als besonders vorteilhaft, wenn das eine Lumen als Spüllumen dienen soll, da solchenfalls eine vollständige Spülung der Lumen sichergestellt werden kann, ohne dass sich Ablagerungen an nicht oder schwer durchströmbaren Totraumstellen bilden können. Die Ausbildung dieser Kommunikation an den distalen Enden der Lumen durch Befestigen und Eindichten der Abschlusskappe stellt eine sehr wirtschaftliche Möglichkeit der Herstellung des Kopplungskörpers bzw. der Anschlussvorrichtung dar. Es sei an dieser Stelle erwähnt, dass die Abschlusskappe selbst in einem Spritzgießverfahren als Spritzgussteil hergestellt werden kann.

Vorteilhafterweise sind das Leitungsmittel und der Kopplungskörper und vorzugsweise auch die Abschlusskappe aus einem biegsamen elastomeren Material, insbesondere aus Silikon oder auf Silikonbasis, ausgebildet, das eine Shore A - Härte von höchstens 65, insbesondere von höchstens 60, insbesondere von höchstens 50, insbesondere von höchstens 40, und weiter von wenigstens 10, insbesondere von wenigstens 15 aufweist.

Die Abschlusskappe ist vorteilhafterweise mit dem spritzgegossenen Material des Kopplungskörpers stoffschlüssig verbunden, insbesondere und vorzugsweise verklebt. Die Abschlusskappe schließt den ersten Bereich des Kopplungskörpers vorzugsweise verrundet ab. Ein Übergang zur Abschlusskappe ist vorzugsweise im Wesentlichen fugen- und stufenfrei.

Zur Perfektionierung des Anfügens der Anschlusskappe wird in weiterer Ausbildung der Erfindung vorgeschlagen, dass der Kopplungskörper einen vorzugsweise dünnen Randbereich aufweist, in welchen die Abschlusskappe eingesetzt ist und der die Abschlusskappe in einer Umfangsrichtung kragenartig umgibt. Ein solcher Randbereich, der eine beispielhafte Wanddicke von 0,2 bis 1,5 mm aufweist, lässt sich im Spritzgießverfahren sehr einfach herstellen, zumal er nur einige wenige Millimeter, beispielsweise 1 bis 10 mm, insbesondere 1 bis 8 mm, insbesondere 1 bis 5 mm hervorzustehen braucht, um eine kragenartige Aufnahme für die Abschlusskappe zu bilden.

Es erweist sich weiter als vorteilhaft, wenn der Kopplungskörper mit einem ersten Bereich, welcher einen den Endabschnitt des Leitungsmittels zumindest teilweise umgebenden Teil, einen daran gegebenenfalls anschließenden Zwischenteil und die Abschlusskappe umfasst, und mit einem demgegenüber mit einer geringeren Dicke ausgebildeten und flächenhaft erstreckten zweiten Bereich ausgebildet ist. Der erwähnte Zwischenteil des Kopplungskörpers ergibt sich dann, wenn der Endabschnitt des Leitungsmittels sich nicht über im Wesentlichen die gesamte Längserstreckung des ersten Bereichs erstreckt, sondern nur einen verhältnismäßig kurzen Ansatz zum Anspritzen des Kopplungskörpers bildet. Solchenfalls werden die Lumen des Leitungsmittels im Inneren des Kopplungskörpers fortgesetzt durch im Zuge des Spritzgießens gebildete kanalbildende Ausnehmungen, mithin Lumen. Einer Ausbildung des Kopplungskörpers mit einem verhältnismäßig kurzen den Endabschnitt des Leitungsmittels umschließenden Teil und einem sich hieran anschließenden Zwischenteil, in dem die Lumen des Leitungsmittels fortgesetzt werden, wird der Vorzug gegeben. Solchenfalls erweist es sich als vorteilhaft, wenn der Endabschnitt des Leitungsmittels, also der mit Material des Kopplungskörpers umspritzte Bereich des Leitungsmittels, etwa 2 bis 20 mm, insbesondere 2 bis 15 mm und weiter insbesondere 3 bis 12 mm und weiter insbesondere 5 bis 10 mm in den Kopplungskörper hinein erstreckt ist.

Es erweist sich weiter als zweckmäßig, wenn der vorerwähnte flächenhaft erstreckte zweite Bereich des Kopplungskörpers mit einer Dicke von 0,1 bis 2 mm, insbesondere 0,1 bis 1,5 mm, insbesondere 0,1 - 1,0 mm, insbesondere von 0,1 - 0,8 mm, insbesondere von 0,1 - 0,5 mm ausgebildet ist.

Zweckmäßigerweise ist der zweite Bereich derart flächenhaft ausladend ausgebildet, dass eine Anlagefläche des zweiten Bereichs an die wundabgewandte Oberseite des Unterdruckverbands wenigstens 1,5 mal so groß, insbesondere wenigstens 1,8 mal so groß ist wie die Anlagefläche des ersten Bereichs. Die Anlagefläche wird dabei in der senkrechten Projektion auf die Erstreckungsebene des Kopplungskörpers betrachtet bzw. berechnet.

Nach einem weiteren Gedanken wir vorgeschlagen, dass der erste Bereich des Kopplungskörpers die Längserstreckung des Leitungsmittels fortsetzt und der zweite Bereich des Kopplungskörpers den ersten Bereich beidseits seiner Längserstreckung flankiert und in einer Draufsicht insbesondere schmetterlingsartig geformt ist, d.h. beidseits der Längserstreckung eine Einschnürung aufweist.

Weiter erweist es sich als vorteilhaft, wenn die maximale Dicke des ersten Bereichs des an das Leitungsmittel angespritzten Kopplungskörpers höchstens 3 mm, insbesondere höchstens 2 mm größer ist als die Dicke des Leitungsmittels und dass die Breite des ersten Bereichs des Kopplungskörpers höchstens 5 mm, insbesondere höchstens 3 mm, insbesondere höchstens 2 mm größer ist als die Breite des Leitungsmittels.

Weiter erweist es sich als besonders vorteilhaft, wenn die Dickenerstreckung des Leitungsmittels höchstens 7 mm, insbesondere höchstens 6 mm, insbesondere wenigstens 3 mm und weiter insbesondere 4 - 6 mm beträgt, wobei seine Breite quer zur Längserstreckung wenigstens 12 mm, insbesondere wenigstens 15 mm, insbesondere zwischen 15 mm und 30 mm, insbesondere zwischen 15 mm und 25 mm, insbesondere zwischen 15 mm und 22 mm, beträgt.

Es kann sich als vorteilhaft erweisen, wenn bei einer mehrlumigen Ausbildung des Leitungsmittels das eine Lumen wesentlich größer als das andere Lumen ausgebildet ist. Das mit einem größeren Leitungsquerschnitt ausgebildete Lumen eignet sich solchenfalls als mit Unterdruck beaufschlagbares Sauglumen zum Abführen von Wundsekreten mit darin oftmals enthaltenen partikelförmigen Bestandteilen. Das andere mit einem kleineren Leitungsquerschnitt ausgebildete Lumen eignet sich als Spüllumen oder zum Heranführen von insbesondere mit Wirkstoffen versetzten fluiden Medien.

Um ein Kollabieren eines insbesondere flach bauenden Leitungsmittels zu verhindern, kann es sich als vorteilhaft erweisen, im Inneren eines Lumens stützende Mittel zum Verhindern des Kollabierens vorzusehen, die sich insbesondere einstückig von Innenwänden des Leitungsmittels in das Innere erstrecken und so Teilquerschnitte eines Lumens definieren oder begrenzen können. Solchenfalls kann es sich gleichwohl als vorteilhaft erweisen, dass zwei innerhalb des bereitgestellten Leitungsmittels gegeneinander nicht unterdruckdicht abgetrennte Lumen oder Teilquerschnitte eines Lumens in Verlängerung innerhalb des Kopplungskörpers quer zu ihrer Längserstreckung druckdicht gegeneinander abgeschlossen sind. Es werden also nach diesem weiteren Erfindungsgedanken innerhalb des Kopplungskörpers voneinander separate Lumen gebildet, die aber gleichwohl an ihren distalen Enden miteinander strömungsmäßig verbunden sein können, sofern dies als zweckmäßig angesehen wird. Hierdurch wird die Stabilität des Kopplungskörpers erhöht und eine wirksame Kollabierverhinderung geschaffen.

Es erweist sich als besonders vorteilhaft, wenn der Kopplungskörper drei nebeneinander erstreckten Lumen umfasst, die an ihren Enden strömungsmäßig miteinander verbunden sind, wobei ein Lumen als Spüllumen ausgebildet ist und die zwei anderen Lumen als Sauglumen ausgebildet sind. Weiter kann es sich als besonders vorteilhaft erweisen, wenn das als Spüllumen ausgebildete Lumen in Richtung auf die Wunde abgeschlossen, also ohne Öffnungen ausgebildet wird, und dann vorzugsweise erst über sein distales Ende mit den weiteren Lumen innerhalb des Kopplungskörpers strömungsmäßig verbunden ist. Dies kann in Weiterbildung der Erfindung - wie eingangs erwähnt - vorteilhafterweise durch eine Strömungskommunikation über die Abschlusskappe realisiert werden.

Zum Anfügen der Anschlussvorrichtung an den Unterdruckverband erweist es sich als vorteilhaft, dass eine dem Unterdruckverband zugewandte Seite des Kopplungskörpers mit einer klebenden Schicht oder einer klebenden Beschichtung ausgebildet ist, um zum bestimmungsgemäßen Gebrauch eine klebende und gegen die Umgebung im wesentlichen unterdruckdichte Verbindung zu dem Unterdruckverband herzustellen.

Weiter erweist es sich als vorteilhaft, dass die klebende Schicht oder Beschichtung von einer wenigstens dreilagigen Haftvermittlungsschicht gebildet ist, die eine mittlere Traglage, eine an der Traglage gehaltene und dem Kopplungskörper zugewandte erste Kleberschicht und eine an der Traglage gehaltene und von dem Kopplungskörper abgewandte zweite Kleberschicht umfasst. Die Haftvermittlungsschicht ist so ausgebildet, dass sie die wenigstens eine Öffnung in dem Kopplungskörper nicht blockiert und dass die erste Kleberschicht und die zweite Kleberschicht von unterschiedlichen Klebematerialien mit unterschiedlichen Klebeeigenschaften gebildet sind. Hierbei erweist es sich als vorteilhaft, dass bei einer Ausbildung des Kopplungskörpers aus Silikon die erste Kleberschicht einen Silikonkleber umfasst. Weiter erweist es sich zumeist als vorteilhaft, wenn die zweite Kleberschicht einen Acrylatkleber umfasst, der dann in aller Regel geeignet ist, mit typischen Wundverbandmaterialien eine im Wesentlichen unterdruckdichte Verbindung einzugehen. Die erste und die zweite Kleberschicht haben vorzugsweise eine Dicke von 20 bis 400 µm. Bei der mittleren Traglage wird ein Vliesmaterial, ein Flachmaterial mit einer textilen Bindung, wie z. B. ein Gestrick, Gewirk oder Gewebe, oder eine Kunststofffolie, eine Metallfolie oder ein Verbundwerkstoff hieraus bevorzugt. Weiter erweist es sich als vorteilhaft, wenn die wundzugewandte Seite der zweiten Kleberschicht von einer ablösbaren Schutzschicht überfangen ist, die vorzugsweise zweiteilig und weiter vorzugsweise mit einer Anfasslasche und/oder einem über die zweite Kleberschicht überstehenden und ergreifbaren Bereich ausgestattet wird.

Nach einem an sich unabhängigen Erfindungsgedanken ist am wundabgewandten Ende des Leitungsmittels, also an demjenigen einer unterdruckerzeugenden Einrichtung zugewandten Ende des Leitungsmittels, ein Verbindungsstück, vorzugsweise aus einem biegsamen polymeren Material, unterdruckdicht und vorzugsweise stoffschlüssig, insbesondere durch eine Klebeverbindung befestigt. Dieses Verbindungsstück kann entweder zum Verbinden mit einem weiteren Leitungsmittelabschnitt dienen oder es dient als Bindeglied zu einem weiteren Kupplungselement, insbesondere Schnellkupplungselement oder Steckverbinder.

Das vorerwähnte Verbindungsstück wird vorzugsweise aus einem biegsamen polymeren Material gebildet, und es wird vorgeschlagen, dass dabei ein einstückig daran anschließendes und somit verliersicher gehaltenes Verschlussmittelteil gebildet wird. Dieses Verschlussmittelteil kann einen oder mehrere Verschlussstopfenabschnitte aufweisen und Öffnungen des Verbindungsstücks oder eines daran anordenbaren Schnellkupplungselements klemmschlüssig und lösbar verschließen. Die Anschlussvorrichtung umfasst vorteilhafterweise ein solches aus biegesteifem Material, insbesondere aus duroplastischem Material, gebildetes Schnellkupplungselement, welches mit dem Verbindungsstück klemmschlüssig verbunden oder klemmschlüssig verbindbar ist.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der Erfindung. In der Zeichnung zeigt:
Figur 1 eine Draufsicht auf eine erfindungsgemäße Anschlussvorrichtung zur Verwendung bei der Unterdrucktherapie von Wunden zur weiteren Verbindung mit einer nicht dargestellten unterdruckerzeugenden Einrichtung;
Figur 2 einen Querschnitt durch ein Leitungsmittel der Anschlussvorrichtung nach Figur 1;

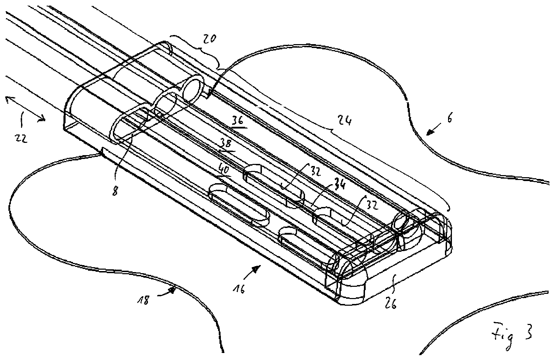

Figur 3 eine vergrößerte perspektivische Teilansicht der Anschlussvorrichtung nach Figur 1 mit Blick auf die wundabgewandte Seite der Anschlussvorrichtung;
Figur 4 eine perspektivische Ansicht entsprechend Figur 3, jedoch ohne eine Abschlusskappe;
Figur 5 eine perspektivische Ansicht der Anschlussvorrichtung in Richtung auf die wundzugewandte Seite der Anschlussvorrichtung, jedoch ohne Leitungsmittel und ohne Abschlusskappe; und
Figur 6 eine perspektivische Ansicht der Abschlusskappe.

Die Figuren zeigen eine insgesamt mit dem Bezugszeichen 2 bezeichnete erfindungsgemäße Anschlussvorrichtung zur Verwendung bei der Unterdrucktherapie von Wunden. Hierfür ist die Anschlussvorrichtung 2 auf noch näher zu beschreibende Weise mit einer nicht dargestellten unterdruckerzeugenden Einrichtung verbindbar, die typischerweise mit einem Flüssigkeitsaufnahmebehälter zum Abscheiden und Sammeln von aus dem Wundraum abgesaugten Wundsekreten und Spülmedien zusammenwirkt. Die Anschlussvorrichtung dient zum unterdruckdichten Ankoppeln an einen die Wunde wiederum im Wesentlichen unterdruckdicht gegen die Umgebung abschließenden Unterdruckverband, wobei eine Unterdruckkommunikation zum Wundraum hergestellt wird.

Die erfindungsgemäße Anschlussvorrichtung 2 umfasst ein insgesamt mit dem Bezugszeichen 4 bezeichnetes Leitungsmittel und einen Kopplungskörper 6. Der Kopplungskörper 6 ist auf noch näher zu beschreibende erfindungsgemäße Weise an einen wundseitigen Endabschnitt 8 des Leitungsmittels in einem Kunststoff-Spritzgießverfahren angespritzt.

An einem wundabgewandten Ende 10 des Leitungsmittels 4 ist beispielhaft ein Verbindungsstück 12 und ein Schnellkupplungselement 14 zum weiteren Anschluss in Richtung auf die nicht dargestellte unterdruckerzeugende Einrichtung vorgesehen.

Der Kopplungskörper 6 umfasst einen im beispielhaft dargestellten Fall blockförmig anmutenden ersten Bereich 16, der die Längserstreckung des Leitungsmittels 4 fortsetzt, und einen demgegenüber mit einer wesentlich geringeren Dicke ausgebildeten und flächenhaft erstreckten zweiten Bereich 18, welcher sich ausgehend von dem ersten Bereich 16 vorzugsweise beidseitig und weiter vorzugsweise auf wenigstens drei Seiten des ersten Bereichs 16 flächenhaft wegerstreckt, damit eine große Anlagefläche des Kopplungskörpers 6 an einen nicht dargestellten Unterdruckverband realisiert werden kann.

Der erste Bereich 16 des Kopplungskörpers 6 umfasst einen den Endabschnitt 8 des Leitungsmittels 4 zumindest teilweise umgebenden Teil 20, einen daran in einer Längsrichtung 22 anschließenden Zwischenteil 24 und eine Abschlusskappe 26. Der den Endabschnitt umgebende Teil 20, der Zwischenteil 24 und der flächenhaft erstreckte zweite Bereich 18 des Kopplungskörpers 6 sind einstückig aus einem biegsamen elastomeren Material, vorzugsweise aus Silikon oder auf Silikonbasis, hergestellt. Hierfür wird das Leitungsmittel 4 bzw. der wundseitige Endabschnitt 8 des Leitungsmittels 4 in eine nicht dargestellte Spritzgießform eingebracht, und das den Kopplungskörper 6 bildende elastomere Material wird an den Endabschnitt 8 des Leitungsmittels 4 angespritzt. Hierbei ist die Spritzgießform derart ausgebildet bzw. ausgestattet, dass das Strömungskanäle bildende Lumen 28, 30 des Leitungsmittels 4 eine Fortsetzung innerhalb des Kopplungskörpers 6 finden. Es müssen im Spritzgießwerkzeug also entsprechende hohlraum- oder kanalbildende Mittel, insbesondere in Form von Stangen oder dergleichen, vorgesehen werden. Obschon nicht dargestellt, wäre es auch denkbar, dass sich das Leitungsmittel 4 bzw. dessen Endabschnitt 8 bis zu der Abschlusskappe 26 erstreckt. Solchenfalls wäre der den Endabschnitt 8 des Leitungsmittels 4 zumindest teilweise umgebende Teil 20 in Längsrichtung 22 länger ausgebildet und der Zwischenteil 24 wäre entbehrlich oder sehr viel kürzer ausgebildet. Es hat sich indessen als vorteilhaft erwiesen, wenn das Leitungsmittel 4 nur mit einem verhältnismäßig kurzen Endabschnitt 8 in den Kopplungskörper 6 einragt, da solchenfalls die Gestaltungsfreiheit des Kopplungskörpers 6 innerhalb des Zwischenteils 24 größer ist und bereits beim Spritzgießvorgang Öffnungen 32 in einer dem Unterdruckverband zugewandten Wandung 34 des Kopplungskörpers 6 ausgebildet werden können.

Der Zwischenteil 24 wird also in seinem Inneren mit in

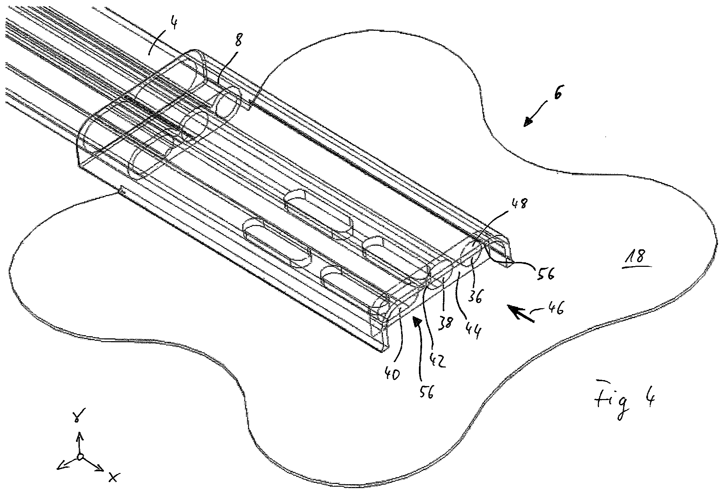

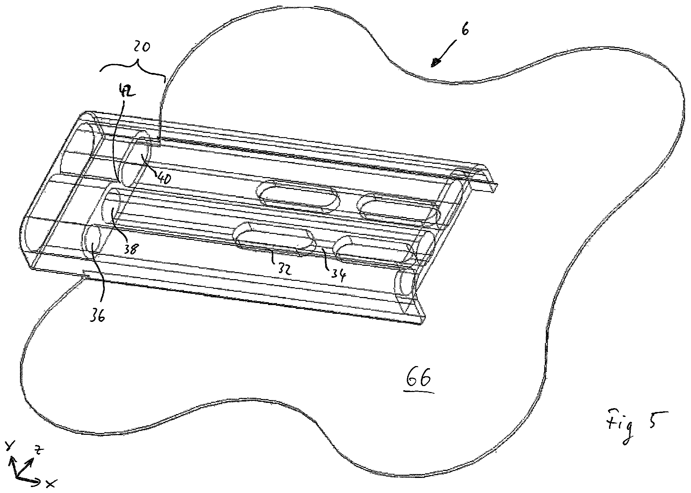

quer zur Längsrichtung 22 voneinander abgetrennten Lumen 36, 38, 40 ausgebildet, die sich wie aus den Figuren ersichtlich an die Lumen 28, 30 des Leitungsmittels 4 anschließen. Dabei ist die beispielhafte Besonderheit gegeben, dass das Lumen 30 des Leitungsmittels 4 mit einem sehr viel größeren Querschnitt als das Lumen 28 ausgebildet ist; es umfasst gewissermaßen zwei Teilquerschnitte, die mit den Bezugszeichen 30a und 30b bezeichnet sind, jedoch nicht strömungsmäßig voneinander getrennt sind. Sie werden durch Längsrippen 41 im Inneren des Leitungsmittels 4 begrenzt, die ein Kollabieren des Lumens 30 verhindern. Die sich an das Lumen 30 bzw. die Teilquerschnitte 30a, 30b des Leitungsmittels 4 anschließenden Lumen 38 und 40 innerhalb des Zwischenteils 24 des Kopplungskörpers 6 sind jedoch durch eine vom Material des Zwischenteils 24 gebildete Wandung 42 voneinander getrennt (was am besten aus Figuren 4 und 5 zu ersehen ist). Diese getrennte Ausbildung ist zwar bevorzugt, jedoch nicht zwingend. Sie bringt den Vorteil mit sich, dass hierdurch die Stabilität des Kopplungskörpers 6 gegen Kollabieren seiner Lumen 36, 38, 40 erhöht wird und dass seine Spülbarkeit mit Spülmedien verbessert wird.

Figur 4 zeigt das Leitungsmittel 4 mit dem angespritzten in sich also einstückigen Kopplungskörper 6 nach dem Spritzvorgang, also noch ohne Abschlusskappe 26. Figur 5 zeigt den Kopplungskörper 6 wiederum ohne Abschlusskappe und ohne Leitungsmittel 4, also in einem an sich nicht existenten Zustand nur zur Verdeutlichung des den Endabschnitt 8 des Leitungsmittels 4 umgebenden Teils 20 des Kopplungskörpers.

Man erkennt weiter am besten aus Figur 4, dass die Lumen 36, 38, 40 des Kopplungskörpers an einer der Abschlusskappe 26 zugewandten Stirnseite 44 des Zwischenteils 24 frei ausmünden. Sie werden durch Anfügen und unterdruckdichtes Befestigen der Abschlusskappe 26 zur Umgebung hin unterdruckdicht abgeschlossen. Die Abschlusskappe 26, die klemmschlüssig oder vorzugsweise stoffschlüssig unlösbar, insbesondere mittels Kleber, befestigt wird, komplettiert den Kopplungskörper 6 der erfindungsgemäßen Anschlussvorrichtung 2. Wie weiter am besten aus Figur 4 ersichtlich ist, wird beim Anspritzen des den Kopplungskörper 6 bildenden elastomeren Materials ein entgegen einer Aufsteckrichtung 46 der Abschlusskappe 26 erstreckter Randbereich 48 gebildet, der vorzugsweise verhältnismäßig dünn, beispielhaft nur 0,5 bis 1,5 mm dick ausgebildet ist und eine Aufnahmeöffnung 50 für die Abschlusskappe 26 begrenzt. Im beispielhaft dargestellten Fall erstreckt sich der Randbereich 48 über drei Seiten, so dass die Aufnahmeöffnung 50 auf der vierten Seite von dem flächenhaft erstreckten zweiten Bereich 18 des Kopplungskörpers 6 begrenzt wird.

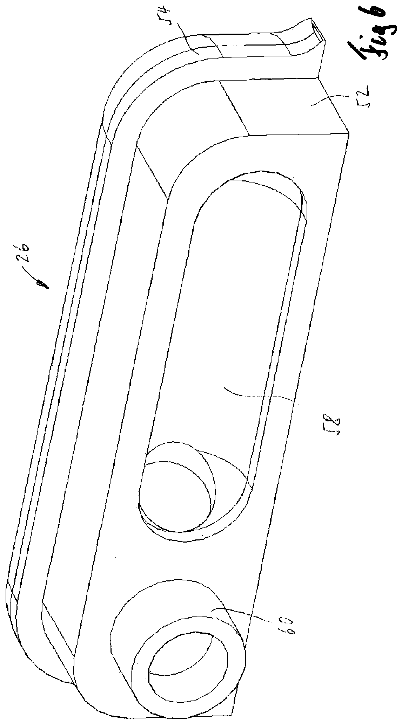

Die vorzugsweise aus demselben Material wie der Kopplungskörper 6 gebildete Abschlusskappe 26 ist in perspektivischer Ansicht in Figur 6 dargestellt. Man erkennt einen Eingriffsabschnitt 52, mit dem die Abschlusskappe 26 in die Aufnahmeöffnung 50 eingreift, und einen von außen sichtbaren Abschnitt 54, der den Kopplungskörper 6 nach außen sichtbar komplettiert. Die Abschlusskappe 26 ist vorzugsweise, jedoch nicht zwingend, derart ausgebildet, dass der sichtbare Abschnitt 54 gegenüber dem Eingriffsabschnitt 52 gerade um die Dicke des vorstehenden Randbereichs 48 des Zwischenteils 24 vorsteht. Auf diese Weise liegt die Abschlusskappe 26 auf Stoß gegen eine Stirnseite 56 des Randbereichs 48 an. Hierdurch wird eine weitere labyrinthartige Abdichtung bewirkt, und außerdem ist ein im Wesentlichen fugenfreier und stufenfreier Übergang von dem Zwischenteil 24 zur Abschlusskappe 26 realisierbar.

Die Abschlusskappe 26 ist weiterhin mit einer insgesamt mit dem Bezugszeichen 58 bezeichneten Ausnehmung ausgebildet, mittels derer eine Strömungskommunikation zwischen wenigstens zwei, im beispielhaft dargestellten Fall zwischen allen drei Lumen 36, 38, 40 erreicht wird, und zwar ausgehend von deren distal mündenden Öffnungen im Bereich der Stirnseite 44. Die Ausnehmung 58 könnte in mehrfacher Weise, im einfachsten Fall beispielsweise in Form einer langlochförmigen Vertiefung realisiert sein, die sich dann an die Stirnseite 44 des Zwischenteils 24 anschließt. Im beispielhaft konkret dargestellten Fall ist die Abschlusskappe mit einem tubusförmigen Ansatz 60 ausgebildet, der beim Aufstecken der Abschlusskappe 26 in das komplementär hierzu ausgebildete Lumen 36 in dem Zwischenteil 24 dichtend eingreift. Dieser tubusförmige Ansatz mündet im Inneren der Abschlusskappe 26 in die quer verlaufende Ausnehmung 58, die sich zur Stirnseite 44 des Zwischenteils 24 langlochförmig öffnet. Auf diese Weise besteht eine Strömungskommunikation zwischen allen Lumen 36, 38, 40.

Es erweist sich im beispielhaft dargestellten Fall als vorteilhaft, dass das Lumen 28 des Leitungsmittels 4 und das daran anschließende Lumen 36 innerhalb des Kopplungskörpers 6 als Spüllumen zur Zuführung eines fluiden Mediums in Richtung auf die Wunde ausgebildet sind. Spülflüssigkeit oder sonstige Fluide, insbesondere Luft, werden auf diese Weise an die distalen Mündungsöffnungen der Lumen 38, 40 in der Stirnseite 44 des Zwischenteils 24 herangeführt. Hierdurch wird eine totraumfreie Spülung der Lumen möglich; es gibt also keinen Leitungsabschnitt, der nicht unmittelbar entlang des Strömungswegs von der Strömung erfasst würde, was im Hinblick auf eine bestimmungsgemäße Funktion der Anschlussvorrichtung und der mit ihr durchgeführten Unterdruckwundtherapie und im Hinblick auf eine Reduzierung von bakteriellem Wachstum und von Infektionen als besonders wertvoll angesehen wird. Der Abschlusskappe 26 kommt also eine Doppelfunktion zu, nämlich zum einen komplettiert sie den Kopplungskörper 6 und verschließt die nach dem Spritzgießverfahren ausmündenden Öffnungen der Lumen 36, 38, 40, und zum anderen wird durch die Abschlusskappe 26 eine Strömungskommunikation zwischen den Lumen 36, 38, 40 realisiert.

Zur Unterdruckkommunikation mit dem Wundraum sind die schon erwähnten Öffnungen 32 in der dem Unterdruckwundverband zugewandten Wandung 34 des Kopplungskörpers 6 ausgebildet. Man erkennt aus den Figuren, dass das als Spüllumen fungierende Lumen 36 des Kopplungskörpers nicht über eine solche Öffnung verfügt, was bevorzugt, jedoch nicht zwingend ist. Die im Bereich der Längserstreckung der Lumen 38, 40 vorgesehenen Öffnungen 32 sind in bevorzugter Weise langlochförmig ausgebildet und haben eine beispielhaft bevorzugte Länge von 8 bis 12 mm. Im beispielhaft dargestellten Fall sind entlang der Erstreckung jedes Lumens 38, 40 zwei solcher langlochförmiger Öffnungen ausgebildet. Im Betrieb kommunizieren die Lumen 38, 40 des Kopplungskörpers über diese Öffnungen 32 und über wenigstens eine Öffnung in dem Unterdruckverband hindurch mit dem Wundraum. Typischerweise werden die Lumen 38, 40 des Kopplungskörpers 6 über das Lumen 30 in dem Leitungsmittel 4 mit Unterdruck beaufschlagt; die Lumen 38, 40 fungieren also typischerweise als Sauglumen zum Anlegen von Unterdruck und zum Abführen von Wundsekreten, Spülflüssigkeit oder sonstigen zugeführten fluiden Medien.

Es wird nun die in den Figuren beispielhaft dargestellte Gestalt des flächenhaft erstreckten zweiten Bereichs 18 des Kopplungskörpers 6 beschrieben. Diese Gestalt oder Form lässt sich in der Draufsicht am ehesten als schmetterlingsförmig bezeichnen, da dieser zweite Bereich 18 in Längsrichtung 22 betrachtet beidseits eine Einschnürung 62 aufweist. Auf diese Weise sind die Abmessungen des Kopplungskörpers 6 bzw. seines zweiten Bereichs 18 entlang von angedeuteten Diagonalen 64, die einen Winkel von etwa 45° zur Längsrichtung 22 bilden, größer als in der Längsrichtung 22 und senkrecht hierzu. Im beispielhaft und bevorzugt dargestellten Fall erstreckt sich der flächenhaft erstreckte dünne zweite Bereich 18 nach drei Seiten über den ebenfalls eher flach bauenden, jedoch als blockförmig zu bezeichnenden ersten Bereich 16 des Kopplungskörpers 6 hinaus. Auf diese Weise ist eine sehr gute Anbindung an einen nicht dargestellten Unterdruckwundverband erreichbar, da die auftretenden Kräfte über eine große Fläche gleichmäßig verteilt und daher ohne lokale Kraftspitzen in diejenigen die Wunde umgebenden Bereiche des Wundverbands und der Körperoberfläche des Patienten eingeleitet werden.

Zur Anbindung an den Unterdruckverband umfasst der Kopplungskörper auf seiner dem Unterdruckverband zugewandten Seite 66 eine nicht dargestellte klebende Schicht. Diese klebende Schicht ist bevorzugtermaßen als eine wenigstens dreilagige Haftvermittlungsschicht ausgebildet, die eine mittlere Traglage, eine an der Traglage gehaltene und dem Kopplungskörper zugewandte erste Kleberschicht und eine an der Traglage gehaltene und von dem Kopplungskörper abgewandte zweite Kleberschicht umfasst. Die Kleberschichten sind jeweils im Hinblick auf die Ausbildung einer Haftverbindung mit dem Material des Kopplungskörpers bzw. mit dem Material des Unterdruckverbands optimiert ausgebildet. Hinsichtlich weiterer Merkmale dieser wenigstens dreilagigen Haftvermittlungsschicht wird Bezug genommen auf die nicht vorveröffentlichte DE 10 2011 108 726.9.

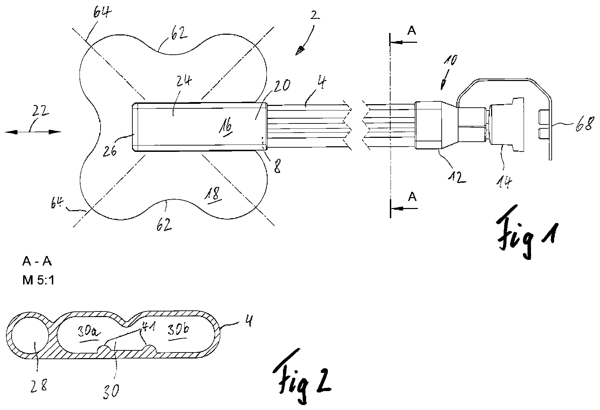

Schließlich zeigt Figur 1 am wundabgewandten Ende 10 des Leitungsmittels das Verbindungsstück 12, das vorzugsweise aus einem biegsamen polymeren Material ausgebildet und unterdruckdicht und vorzugsweise stoffschlüssig an das wundabgewandte Ende 10 des Leitungsmittels 4 angefügt ist. Das Verbindungsstück 12 umfasst ein vorzugsweise einstückig daran anschließendes und somit verliersicher gehaltenes Verschlussmittelteil 68, welches Öffnungen des Verbindungsstücks 12 oder eines daran anordenbares Schnellkupplungselements 14 klemmschlüssig und lösbar zu verschließen vermag. Bei dem Verbindungsstück 12 handelt es sich vorzugsweise um ein einstückig samt Verschlussmittelteil 68 hergestelltes Spritzgussteil. An das Schnellkupplungselement 14 lässt sich ein weiterer zu der nicht dargestellten unterdruckerzeugenden Einrichtung nebst Flüssigkeitssammelbehälter führender weiterer Leitungsmittelabschnitt über ein komplementär ausgebildetes Schnellkupplungselement unterdruckdicht anschließen.

## Patentansprüche

1. Anschlussvorrichtung (2) zur Verwendung bei der Unterdrucktherapie von Wunden, mit einem mit Unterdruck und/oder fluiden Medien beaufschlagbaren biegsamen Leitungsmittel (4) mit wenigstens zwei Lumen (28, 30) und mit einem mit dem Leitungsmittel verbundenen Kopplungskörper (6), wobei der Kopplungskörper (6) auf einen die Wunde überfangenden und gegen die Atmosphäre dichtend abschließenden Unterdruckverband aufbringbar ist, wobei das Leitungsmittel (4) durch wenigstens eine Öffnung (32) hindurch mit dem Wundraum kommuniziert, wobei der Kopplungskörper aus einem elastomeren biegsamen Material ausgebildet ist, **dadurch gekennzeichnet, dass** das elastomere biegsame Material unlösbar an einen wundseitigen Endabschnitt (8) des Leitungsmittels (4) angespritzt ist, dass zur weiteren Ausbildung des Kopplungskörpers (6) an das angespritzte Material eine Abschlusskappe (26) angefügt und vorzugsweise unlösbar befestigt ist, wodurch der Kopplungskörper (6) nach außen dicht abgeschlossen ist und dass der Kopplungskörper (6) so ausgebildet ist, dass nach dem Anspritzen wenigstens zwei Lumen (36, 38, 40) frei ausmünden, und dass durch Befestigen der Abschlusskappe (26) die frei ausmündenden Lumen (36, 38, 40) nach außen dicht abgeschlossen und miteinander durchströmbar verbunden werden, indem die Abschlusskappe (26) eine die beiden Lumen miteinander verbindende Ausnehmung (58) aufweist.

2. Anschlussvorrichtung nach Anspruch 1, **dadurch gekennzeichnet, dass** das Leitungsmittel (4) im wesentlichen parallel zu der Ebene der flächenhaften Erstreckung des Kopplungskörpers (6) an den Kopplungskörper (6) herangeführt ist.

3. Anschlussvorrichtung nach Anspruch 1 oder 2, **dadurch gekennzeichnet, dass** das Leitungsmittel (4) und der Kopplungskörper (6) und vorzugsweise auch die Abschlusskappe (26) aus einem biegsamen elastomeren Material, insbesondere aus Silikon oder auf Silikonbasis, ausgebildet sind, das eine Shore A - Härte von höchstens 65, insbesondere von höchstens 60, insbesondere von höchstens 50, insbesondere von höchstens 40, und weiter von wenigstens 10, insbesondere von wenigstens 15 aufweist.

4. Anschlussvorrichtung nach einem oder mehreren der vorstehenden Ansprüche, **dadurch gekennzeichnet, dass** der Kopplungskörper (6) einen vorzugsweise dünnen Randbereich (48) aufweist, in welchen die Abschlusskappe (26) eingesetzt ist und der die Abschlusskappe (26) in einer Umfangsrichtung kragenartig umgibt.

5. Anschlussvorrichtung nach einem oder mehreren der vorstehenden Ansprüche, **dadurch gekennzeichnet, dass** der Kopplungskörper (6) mit einem ersten Bereich (16), welcher einen den Endabschnitt (8) des Leitungsmittels (4) zumindest teilweise umgebenden Teil (20), einen daran gegebenenfalls anschließenden Zwischenteil (24) und die Abschlusskappe (26) umfasst, und mit einem demgegenüber mit einer geringeren Dicke ausgebildeten und flächenhaft erstreckten zweiten Bereich (18) ausgebildet ist.

6. Anschlussvorrichtung nach einem oder mehreren der vorstehenden Ansprüche, **dadurch gekennzeichnet, dass** der Endabschnitt (8) des Leitungsmittels (4) etwa 2 bis 20 mm, insbesondere 2 bis 15 mm und weiter insbesondere 3 bis 12 mm und weiter insbesondere 5 bis 10 mm in den Kopplungskörper (6) hinein erstreckt ist.

7. Anschlussvorrichtung nach Anspruch 5 oder 6, **dadurch gekennzeichnet, dass** der zweite Bereich (18) des Kopplungskörpers (6) mit einer Dicke von 0,1 - 2,0 mm, insbesondere 0,1 - 1,5 mm, insbesondere 0,1 - 1,0 mm, insbesondere von 0,1 - 0,8 mm, insbesondere von 0,1 - 0,5 mm ausgebildet ist und dass der zweite Bereich (18) derart flächenhaft ausladend ausgebildet ist, dass eine Anlagefläche des zweiten Bereichs (18) an die wundabgewandte Oberseite des Unterdruckverbands wenigstens 1,5 mal so groß, insbesondere wenigstens 1,8 mal so groß ist wie die Anlagefläche des ersten Bereichs (16).

8. Anschlussvorrichtung nach einem oder mehreren der Ansprüche 5 - 7, **dadurch gekennzeichnet, dass** der erste Bereich (16) des Kopplungskörpers (6) die Längserstreckung des Leitungsmittels (8) fortsetzt und der zweite Bereich (18) des Kopplungskörpers (6) den ersten Bereich (16) beidseits seiner Längserstreckung flankiert und in einer Draufsicht schmetterlingsartig geformt ist.

9. Anschlussvorrichtung nach einem oder mehreren der Ansprüche 5-8, **dadurch gekennzeichnet, dass** die maximale Dicke des ersten Bereichs (16) des an das Leitungsmittel (8) angespritzten Kopplungskörpers (6) höchstens 3 mm, insbesondere höchstens 2 mm größer ist als die Dicke des Leitungsmittels (4) und dass die Breite des ersten Bereichs (16) des Kopplungskörpers (6) höchstens 5 mm, insbesondere höchstens 3 mm, insbesondere höchstens 2 mm größer ist als die Breite des Leitungsmittels (4).

10. Anschlussvorrichtung nach einem oder mehreren der vorstehenden Ansprüche, **dadurch gekennzeichnet, dass** die Dickenerstreckung des Leitungsmittels (4) höchstens 7 mm, insbesondere höchstens 6 mm, insbesondere wenigstens 3 mm und weiter insbesondere 4-6 mm beträgt, wobei die Breite quer zur Längserstreckung wenigstens 12 mm, insbesondere wenigstens 15 mm, insbesondere zwischen 15 mm und 30 mm, insbesondere zwischen 15 mm und 25 mm, insbesondere zwischen 15 mm und 22 mm, beträgt.

11. Anschlussvorrichtung nach einem oder mehreren der vorstehenden Ansprüche, **dadurch gekennzeichnet, dass** ein erstes Lumen (28) des Leitungsmittels (4) ein Spüllumen oder Zuführlumen für fluide Medien zur Wunde bildet und dass ein zweites Lumen (30) des Leitungsmittels (4) ein mit Unterdruck beaufschlagbares Sauglumen zum Abführen von fluiden Medien von der Wunde in Richtung auf eine Unterdruck erzeugende Einrichtung bildet, und dass das zweite Lumen (30) einen größeren Querschnitt aufweist als das erste Lumen (28).

12. Anschlussvorrichtung nach einem oder mehreren der vorstehenden Ansprüche, **dadurch gekennzeichnet, dass** der Kopplungskörper (6) drei nebeneinander erstreckte Lumen (36, 38, 40) umfasst, die an ihren Enden strömungsmäßig miteinander verbunden sind, wobei ein Lumen (36) als Spüllumen ausgebildet ist und die zwei anderen Lumen (38, 40) als Sauglumen ausgebildet sind.

13. Anschlussvorrichtung nach einem oder mehreren der vorstehenden Ansprüche, **dadurch gekennzeichnet, dass** das als Spüllumen ausgebildete Lumen (36) in Richtung auf die Wunde abgeschlossen ist und nur über sein distales Ende mit einem oder mehreren anderen Lumen strömungsmäßig verbunden ist.

14. Anschlussvorrichtung nach Anspruch 13, **dadurch gekennzeichnet, dass** das Spüllumen (36) mit einem oder mehreren anderen Lumen (38, 40) nur über die Abschlusskappe (26) strömungsmäßig verbunden ist.

15. Anschlussvorrichtung nach einem oder mehreren der vorstehenden Ansprüche, **dadurch gekennzeichnet, dass** eine dem Unterdruckverband zugewandte Seite des Kopplungskörpers (6) mit einer klebenden Schicht oder einer klebenden Beschichtung ausgebildet ist, um zum bestimmungsgemäßen Gebrauch eine klebende und gegen die Umgebung im wesentlichen unterdruckdichte Anbindung an den Unterdruckverband herzustellen.

## Claims

1. Connection device (2) for use in the negative pressure therapy of wounds with a flexible conducting means (4) which can be impinged with negative pressure and/or fluid media with at least two lumens (28, 30) and with a coupling body (6) which is connected with the conducting means, wherein the coupling body (6) can be placed on a negative pressure bandage which covers the and seals the wound tight against the atmosphere, wherein the conducting means (4) communicates through at least one opening (32) with the wound space, wherein the coupling body is formed from an elastomeric flexible material, **characterized in that** the elastomeric flexible material is non-detachably injection molded onto a wound side end section (8) of the conducting means (4), and **in that** for further configuration of the coupling body (6) an end cap (26) is attached and preferably non-detachably fastened to the injection molded material, thereby sealing the coupling body (6) tight against the outside and that the coupling body (6) is configured so that after the injection molding at least one lumen (36, 38, 40) ends freely and that by fastening the end cap (26) the freely ending lumen (36, 38, 40) is sealed tight towards the outside.

2. Connection device according to claim 1, **characterized in that** the conducting means (4) leads to the coupling body(6) essentially parallel to the plane of the two-dimensional extent of the coupling body (6).

3. Connection device according to claim 1 or 2, **characterized in that** the conducting means (4) in the coupling body (6) and preferably also the end cap (26) are formed from a flexible elastomeric material in particular from silicone or silicone basis which has a Shore-A hardness of at most 65, in particular of the most 60, in particular of at most 50, in particular of at most 40, and further of at least 10 in particular of at least 15.

4. Connection device according to one or more of the preceding claims, **characterized in that** the coupling body (6) has a preferably thin border region (48) into which the end cap (26) is inserted and which surrounds the end cap (26) collar like in a circumferential direction.

5. Connection device according to one or more of the preceding claims, **characterized in that** the coupling body (6) is formed with a first region (16) which includes a part (20) which at least partially surrounds an end section (8) of the conducting means (4) an intermediate part (24) which adjoins the part (20) and the end cap (26), and with a second region (18) which has a smaller thickness relative to the first region and extends two dimensionally.

6. Connection device according to one or more of the preceding claims, **characterized in that** the end section (8) of the conducting means (4) extends into the coupling body (6) by about 2 to 20 mm, in particular 2 to 15 mm, and further in particular 3 to 12 mm and further in particular 5 to 10 mm.

7. Collection device according to claim 5 or 6, **characterized in that** in the second region (18) of the coupling body (6) is configured with a thickness of 0.1 - 2.0 mm, in particular 0.1 - 1.5 mm, in particular 0.1 - 1.0 mm, in particular 0.1 - 0.8mm, in particular of 0.1 - 0.5 mm and that the second region (18) is configured to have a two dimensionally expansive extent so that a contact surface of the second region (18) to the wound averted top side of the negative pressure bandage is at least 1.5 times, in particular at least 1.8 times as great as the contact surface of the first region (16).

8. Connection device according to one or more of the claims 5-7, **characterized in that** the first region (16) of the coupling body (6) continues the longitudinal extent of the conduction means (8) and the second region (18) of the coupling body (6) flanks the first region (16) on both sides of its longitudinal extent and is formed in the shape of a butterfly when viewed from the top.

9. Collection device according to one or more of the claims 5-8, **characterized in that** the maximum thickness of the first region (16) of the coupling body (6) which is injection molded onto the conducting means (8) is at most 3 mm, in particular at most 2 mm greater than the thickness of the conducting means (4) and that the width of the first region (16) of the coupling body (6) is at most 5 mm, in particular at most 3 mm, in particular at most 2 mm greater than the width of the conducting means (4) .

10. Connection device according to one or more of the preceding claims, **characterized in that** the thickness extent of the conducting means (4) is at most 7 mm, in particular at most 6 mm, in particular at least 3 mm, and further in particular 4 - 6 mm, wherein the width transverse to the longitudinal extent is at least 12 mm, in particular at least 15 mm, in particular between 15 mm and 30 mm, in particular between 15 mm and 25 mm, in particular between 15 mm and 22 mm.

11. Collection device according to one or more of the preceding claims **characterized in that** a first lumen (28) of the conducting means (4) forms in rinsing lumen or delivery lumen for fluid media to the wound and that a second lumen (30) of the conducting means (4) is a suction lumen which can be impinged with negative pressure for discharge of fluid media from the wound in the direction towards a negative pressure generating device and that the second lumen (30) has a greater cross-section than the first lumen (28).

12. Connection device according to one or more of the preceding claims **characterized in that** the coupling body (6) includes three adjacently extending lumens (36, 38, 40) which are fluidly connected at their ends, wherein one lumen (36) is configured as rinsing lumen and the two other lumens (38, 40) are configured as suction lumens.

13. Connection device according to one or more of the preceding claims **characterized in that** the lumen which is configured as rinsing lumen (36) is closed in the direction towards the wound and is fluidly collected with one or more other lumens only via its distal end.

14. Collection device according to claim 13, **characterized in that** the rinsing lumen (36) is fluidly connected with one or more other lumens (38, 40) only via the end cap (26) .

15. Connection device according to one or more of the preceding claims **characterized in that** a side of the coupling body (6) which side faces the negative pressure bandage is configured with an adhesive layer or an adhesive coating, in order to, for the intended use of the device create an adhesive connection to the negative pressure bandage which is essentially negative pressure tight.

## Revendications

1. Dispositif de raccordement (2) destiné à être utilisé dans le traitement de plaies par pression négative, comprenant un moyen de conduite (4) souple qui peut être alimenté en pression négative et/ou en milieux fluides et qui comprend au moins deux lumières (28, 30), ainsi qu'un corps d'accouplement (6) relié au moyen de conduite (4), dans lequel le corps d'accouplement (6) peut être appliqué sur un pansement à pression négative qui recouvre la plaie et isole celle-ci de façon étanche par rapport à l'atmosphère, dans lequel ledit moyen de conduite (4) communique par au moins un orifice (32) avec la zone de la plaie, dans lequel le corps d'accouplement est réalisé en un matériau élastomère souple, **caractérisé par le fait que** le matériau élastomère souple est surmoulé par injection de manière inamovible sur une portion d'extrémité côté plaie (8) du moyen de conduite (4), que, pour l'autre formation du corps d'accouplement (6), un capuchon de fermeture (26) est joint et, de préférence, fixé de manière inamovible sur le matériau surmoulé par injection, ce par quoi le corps d'accouplement (6) est fermé de manière étanche vers l'extérieur, et que le corps d'accouplement (6) est réalisé de telle sorte que, après le surmoulage par injection, au moins deux lumières (36, 38, 40) débouchent librement, et que, en fixant ledit capuchon de fermeture (26), les lumières (36, 38, 40) débouchant librement sont fermées de manière étanche vers l'extérieur et sont reliées entre elles de manière à pouvoir être traversées **par le fait que** le capuchon de fermeture (26) présente un évidement (58) reliant entre elles les deux lumières.

2. Dispositif de raccordement selon la revendication 1, **caractérisé par le fait que** le moyen de conduite (4) est amené au corps d'accouplement (6) pour l'essentiel parallèlement au plan de l'extension en nappe du corps d'accouplement (6).

3. Dispositif de raccordement selon la revendication 1 ou 2, **caractérisé par le fait que** le moyen de conduite (4) et le corps d'accouplement (6) et de préférence également le capuchon de fermeture (26) sont réalisés à partir d'un matériau élastomère flexible, en particulier à partir de silicone ou sur la base de silicone, qui présente une dureté Shore A de 65 tout au plus, en particulier de 60 tout au plus, en particulier de 50 tout au plus, en particulier de 40 tout au plus et encore d'au moins 10, en particulier d'au moins 15.

4. Dispositif de raccordement selon l'une ou plusieurs des revendications précédentes, **caractérisé par le fait que** le corps d'accouplement (6) présente une zone marginale (48) de préférence mince dans laquelle le capuchon de fermeture (26) est inséré et qui entoure à la manière d'une collerette ledit capuchon de fermeture (26) dans une direction circonférentielle.

5. Dispositif de raccordement selon l'une ou plusieurs des revendications précédentes, **caractérisé par le fait que** le corps d'accouplement (6) est réalisé avec une première zone (16) qui comprend une partie (20) entourant au moins en partie la portion d'extrémité (8) du moyen de conduite (4), une partie intermédiaire (24) le cas échéant adjacente à cette première ainsi que le capuchon de fermeture (26), et avec une deuxième zone (18) présentant une épaisseur plus faible par rapport à la première et étendue en nappe.

6. Dispositif de raccordement selon l'une ou plusieurs des revendications précédentes, **caractérisé par le fait que** la portion d'extrémité (8) du moyen de conduite (4) s'étend d'environ 2 à 20 mm, en particulier de 2 à 15 mm et plus particulièrement de 3 à 12 mm et plus particulièrement de 5 à 10 mm dans le corps d'accouplement (6).

7. Dispositif de raccordement selon la revendication 5 ou 6, **caractérisé par le fait que** la deuxième zone (18) du corps d'accouplement (6) est configurée avec une épaisseur comprise entre 0,1 et 2,0 mm, en particulier entre 0,1 et 1,5 mm, en particulier entre 0,1 et 1,0 mm, en particulier entre 0,1 et 0,8 mm, en particulier entre 0,1 et 0,5 mm, et que l'étendue en nappe de la deuxième zone (18) est configurée de telle sorte qu'une surface d'appui par laquelle la deuxième zone (18) entre en contact avec la face supérieure du pansement à pression négative laquelle montre dans la direction opposée à la plaie, fait au moins 1,5 fois, en particulier au moins 1,8 fois la taille de la surface d'appui de la première zone (16).

8. Dispositif de raccordement selon l'une ou plusieurs des revendications 5 à 7, **caractérisé par le fait que** la première zone (16) du corps d'accouplement (6) continue l'extension longitudinale du moyen de conduite (8), et que la deuxième zone (18) du corps d'accouplement (6) flanque la première zone (16) de part et d'autre de son extension longitudinale et est formée, vue de dessus, à la manière d'un papillon.

9. Dispositif de raccordement selon l'une ou plusieurs des revendications 5 à 8, **caractérisé par le fait que** l'épaisseur maximale de la première zone (16) du corps d'accouplement (6) surmoulé par injection sur le moyen de conduite (8) est 3 mm tout au plus, en particulier 2 mm tout au plus, plus grande que l'épaisseur du moyen de conduite (4) et que la largeur de la première zone (16) du corps d'accouplement (6) est 5 mm tout au plus, en particulier 3 mm tout au plus, en particulier 2 mm tout au plus, plus grande que la largeur du moyen de conduite (4).

10. Dispositif de raccordement selon l'une ou plusieurs des revendications précédentes, **caractérisé par le fait que** l'étendue en épaisseur du moyen de conduite (4) est de 7 mm tout au plus, en particulier de 6 mm tout au plus, en particulier d'au moins 3 mm, et plus particulièrement comprise entre 4 et 6 mm, la largeur transversale à l'extension longitudinale est d'au moins 12 mm, en particulier d'au moins 15 mm, en particulier comprise entre 15 mm et 30 mm, en particulier comprise entre 15 mm et 25 mm, en particulier entre 15 mm et 22 mm.

11. Dispositif de raccordement selon l'une ou plusieurs des revendications précédentes, **caractérisé par le fait qu'**une première lumière (28) du moyen de conduite (4) forme une lumière de rinçage ou une lumière d'alimentation en milieux fluides à la plaie et qu'une deuxième lumière (30) du moyen de conduite (4) forme une lumière d'aspiration apte à être mise en dépression pour faire évacuer des milieux fluides de la plaie en direction d'un dispositif de génération de pression négative, et que la deuxième lumière (30) présente une section transversale supérieure à celle de la première lumière (28).

12. Dispositif de raccordement selon l'une ou plusieurs des revendications précédentes, **caractérisé par le fait que** le corps d'accouplement (6) comprend trois lumières (36, 38, 40) s'étendant les unes à côtés des autres qui sont reliées fluidiquement entre elles à leurs extrémités, une lumière (36) étant réalisée en tant que lumière de rinçage et les deux autres lumières (38, 40) étant réalisées en tant que lumières d'aspiration.

13. Dispositif de raccordement selon l'une ou plusieurs des revendications précédentes, **caractérisé par le fait que** la lumière (36) réalisée en tant que lumière de rinçage est fermée en direction de la plaie et n'est reliée fluidiquement que par son extrémité distale à une ou plusieurs autres lumières.

14. Dispositif de raccordement selon la revendication 13, **caractérisé par le fait que** la lumière de rinçage (36) n'est reliée fluidiquement que par ledit capuchon de fermeture (26) à une ou plusieurs autres lumières (38, 40).

15. Dispositif de raccordement selon l'une ou plusieurs des revendications précédentes, **caractérisé par le fait qu'**une face du corps d'accouplement (6) laquelle montre vers le pansement à pression négative est réalisée avec une couche adhésive ou un revêtement adhésif pour établir, pour l'utilisation conforme, une liaison au pansement à pression négative qui est adhésive et pour l'essentiel étanche à la pression négative par rapport à l'environnement.
